# NEUE EUROPÄISCHE PATENTSCHRIFT

(11) **EP 0 607 128 B2**
(45) Veröffentlichungstag und Bekanntmachung des Hinweises auf die Entscheidung über den Einspruch: **21.02.2007**
(45) Hinweis auf die Patenterteilung: 17.09.1997
(21) Anmeldenummer: 91914752.0
(22) Anmeldetag: 19.08.1991
(51) Int. Cl.: A61K 31/19, C07C 57/58

(54) **ARZNEIMITTEL SOWIE DEREN HERSTELLUNG UND DEREN VERWENDUNG BEI DER BEKÄMPFUNG VON SCHMERZEN UND/ODER ENTZÜNDUNGEN AN TIEREN UND MENSCHEN**
DRUGS, THEIR MANUFACTURE, AND THEIR USE AS PAINKILLERS AND/OR TO TREAT INFLAMMATIONS IN HUMANS AND ANIMALS
MEDICAMENT, SA PRODUCTION ET SONT UTILISATION COMME ANALGESIQUE ET/OU ANTI-INFLAMMATOIRE POUR ANIMAUX ET ETRES HUMAINS

(30) Priorität: 12.09.1990 DE 4028906
(43) Veröffentlichungstag der Anmeldung: 27.07.1994
(73) Patentinhaber: PAZ Arzneimittel- Entwicklungsgesellschaft mbH, 65933 Frankfurt am Main (DE)
(72) Erfinder: GEISSLINGER, Gerd, D-8500 Nürnberg 1 (DE); BRUNE, Kay, D-8525 Marloffstein (DE)
(74) Vertreter: Wagner, Jutta
(86) Internationale Anmeldenummer: PCT/EP1991/001568
(87) Internationale Veröffentlichungsnummer: WO 1992/004018

(56) Entgegenhaltungen:
- EP-A- 0 223 389
- WO-A-91/06295
- DE-A- 2 809 794
- GB-A- 2 111 493
- US-A- 4 209 638
- US-A- 4 927 854
- Experientia, Band. 47, 1991;, K. Brune et al.: "Aspirin-like drugs may block pain independently of prostaglandin synthesis inhibition ", Seiten 257-261
- Folia pharmacol. japon., Band. 90, 1987; Yamaguchi T. et al.: "The inhibitory activities of 480156-S and its related compounds on prostaglandin synthetase ", Seiten 295-302
- Br. J. clin. Pharmac., Band. 10, 1980; Gerald A. Higgs: "Arachidonic acid metabolism, pain and hyperalgesia: the mode of action of non-steroid mild analgesics ", Seiten 233S-235S
- Clin. Pharmacol. Ther., Band., Februar 1987; A. Sunshine et al.: "Flurbiprofen, flurbiprofen dextrorotatory component (BTS 24332) and placebo in postepisiotomy pain ", Seite 162
- Adams et al, Anti-rheumatic Drugs, 1983, pp.243-263
- Martindale 1989
- J. Biological Chemistry, vol. 260, n° 23, pp. 12572-12578 (1985)
- J. Pharmaceutical Sciences, vol. 77, n° 8, pp. 666-669 (1988)
- J. Pharmacol. and Experimental Therapeutics, vol. 249, n° 2, pp. 378-385 (1989)
- Drugs, 41(4), pp. 533-547 (1991)

## Beschreibung

Gegenstand der vorliegenden Erfindung ist die Verwendung von Flurbiprofen zur Herstellung von Arzneimitteln zur Behandlung von Schmerzen und/oder Entzündungen an Menschen und Tieren, welche bei Schmerzen überwiegend R(-)-Flurbiprofen und bei Entzündungen überwiegend S(+)-Flurbiprofen enthalten. Der Wirkstoff kann aus den zuvor separierten Flurbiprofen-Enantiomeren im gewünschten Verhältnis bestehen.

Flurbiprofen (2-(2-Fluor-4-biphenylyl)-propionsäure) der folgenden Struktur 1 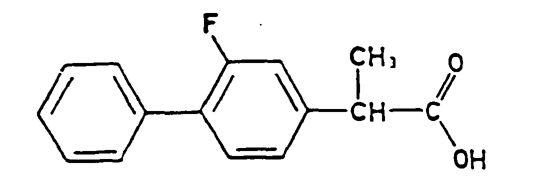 ist ein seit langem bekannter Arzneistoff (DE-C-1518528), welcher wegen seiner anti-inflammatorischen, anti-pyretischen und analgetischen Wirkung in großem Umfang eingesetzt wird (Martindale, The Extra Pharmacopeia, 20. Edit., Seite 18, 1989).

Bei der chemischen Synthese fällt Flurbiprofen normalerweise als Razemat an und wird auch in dieser Form in den verschiedenen Arzneimitteln eingesetzt. Es ist ferner bekannt, daß dieser Stoff, insbesondere bei der Langzeitbehandlung von schmerzhaften und entzündlichen Prozessen erhebliche unerwünschte Nebenwirkungen, insbesondere gastrointestinale Irritationen oder Schädigungen, wie Ulzera, Perforationen, aufweist (vgl. Martindale s.o.).

Da bekannt ist, daß bei vielen pharmakologisch wirksamen 2-Arylpropionsäuren die biologische in vitro Aktivität (Prostaglandinsynthesehemmung, Thrombozytenaggregationshemmung) des einen Enantiomeren größer ist als die des anderen, während die Nebenwirkungen in vielen Fällen beiden Enantiomeren oder sogar dem pharmakologisch weniger wirksamen Enantiomeren zuzuschreiben sind, wird in der DE-A 28 09 794 ein Verfahren vorgeschlagen, ein Anteil der pharmakologisch wirksamen Enantiomeren gegenüber dem Razemat zu erhöhen. Zu diesem Zweck wird eine Lösung, welche den razemischen Wirkstoff oder einen an einem der Enantiomere bereits teilweise angereicherten Wirkstoff enthält, in einem unpolaren Lösungsmittel mit einem optisch aktiven Alkylamin, vorzugsweise α-Phenylethylamin, zu einer diastereomeren Salzmischung umsetzt, wobei die Lösungsmittelmenge nicht ausreicht, um dieses Salz vollständig zu lösen. Die schwere lösliche Komponente reichert sich daher im Niederschlag an. Je nach verwendeter optisch aktiver Base und angewandtem Lösungsmittel lassen sich auf diese Weise unter anderem auch die beiden optischen aktiven Enantiomeren des Flurbiprofens herstellen. Pharmakologische Wirksamkeiten der Enantiomeren sind in dieser Literatur allerdings nicht angegeben.

Aus Sunshine, A. et al., Clin. Pharmakol. Ther. 41(2), 162, 1987 ist es bekannt, das S(+)-Flurbiprofen als Analgetikum bei post Episiotomie-Schmerzen einzusetzen. In einer Doppelblindstudie wurde dabei gefunden, daß S-Flurbiprofen verglichen mit dem Razemat bei halber Dosierung bereits wirksamer ist, so daß angenommen wurde, daß das Enantiomere für die analgetische Wirkung allein verantwortlich ist.

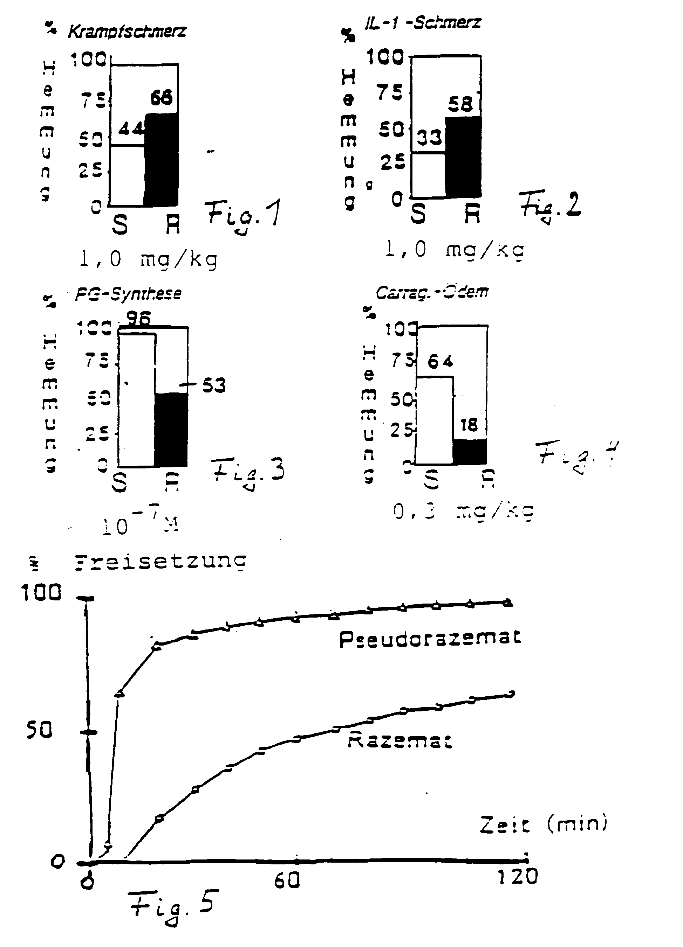

Überraschenderweise wurde nun gefunden, daß entgegen dieser Kenntnis nicht das S(+)-, sondern daß R(-)-Flurbiprofen in anerkannten Schmerzmodellen ausgeprägt analgetisch wirksam ist. Dieses unerwartete Ergebnis wurde durch Untersuchung an zwei Tiermodellen (Maus und Ratte) belegt. Sowohl im Krampfschmerztest an der Maus als auch im Interleukin 1 induzierten Schmerztest bei der Ratte ist das R-Enantiomer, wie aus den Figuren 1 und 2 hervorgeht, um ungefähr ein Drittel bis zur Hälfte wirksamer. Diese Resultate sind auch bei der Ratte, anders als bei anderen bekannten Arylpropionsäuren, den Enantiomeren eindeutig zuzuordnen, da sowohl nach R- als auch nach S-Flurbiprofen-Applikation keine bzw. nur eine sehr geringe Inversion stattfand.

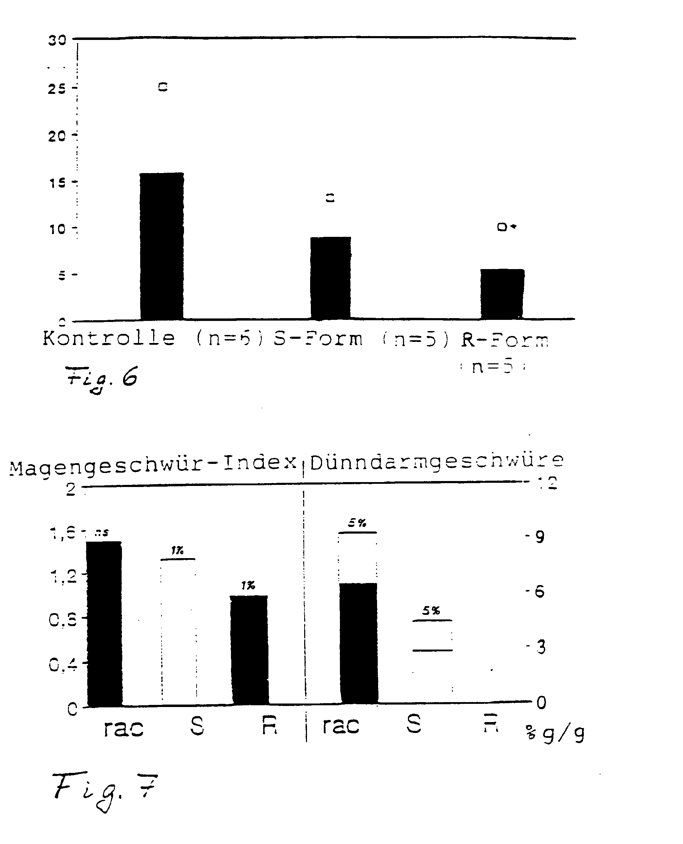

Ferner ist S(+)-Flurbiprofen entgegen dem publizierten Erkenntnisstand (s.o.) bei der Applikation nach Episiotomie bevorzugt antiphlogistisch und nicht vorzugsweise analgetisch wirksam. Dieses gleichfalls überraschende Prüfungsergebnis wurde an zwei unabhängig voneinander gewählten Entzündungsmodellen geprüft. So wurde an Makrophagen (Peritoneum der Maus) bewiesen, daß S(+)-Flurbiprofen die Prostaglandin-Freisetzung ausgeprägter als die R(-)-Form hemmt (Figur 3).

Beim Carrageenin-Pfotenödem der Ratte ist S(-)-Flurbiprofen der R(-)-Form gleichfalls in seiner entzündungshemmenden Wirkung überlegen, wie die Figuren 3 und 4 demonstrieren.

Nach dem gegenwärtigen Wissensstand zum Wirkungsmechanismus von Arzneimitteln bei der erfolgreichen Bekämpfung von Schmerzen unterschiedlicher Genese muß wie folgt differenziert werden.

Bei Analgetika hat der rasche Wirkungseintritt einen herausragenden Stellenwert. Dieser setzt bei oralen, topischen und anderen nicht parenteralen Darreichungsformen zunächst eine beschleunigte Freisetzung sowie hinreichende gute Bioverfügbarkeit des Wirkstoffs oder der Wirkstoffe voraus. Ferner blockieren solche Schmerzmittel, weil die Leitung von Schmerzempfindungen über ein aus der Peripherie zum Zentralen Nervensystem (ZNS) aufsteigendes Leitungssystem erfolgt, die auf verschiedenen Ebenen des ZNS vorhandenen Kontrollmechanismen, an denen Rezeptoren mit chiralen Strukturen beteiligt sind.

Ferner wird angenommen, daß die Hemmung der Prostaglandinbiosynthese als gemeinsames Merkmal des Wirkungsmechanismus bei Analgetika und Antiphlogistika wirkt (Vane, J.R., Nature 231ff, 1971, Higgs, G.A., Brit. J. Clin. Pharmacol. 10, 233ff, 1980). Somit ist diese Wirkung als Bindeglied zwischen der Schmerzlinderung und der Entzündungshemmung zu verstehen. Allerdings lassen sich nicht alle Effekte allein mit diesem Mechanismus erklären. So sind bei sauren Analgetika und/oder Antiphlogistika wie Flurbiprofen auch neurophysiologische Effekte als Folge von Einlagerung solcher Wirkstoffe in Zellmembranen wahrscheinlich.

Prostaglandine sind an der Auslösung klassischer Symptome bei Entzündungen wie Rötung, Schwellung, Ödeme und somit Schmerz beteiligt. Solche entzündliche Veränderungen lassen sich durch entzündungshemmende Wirkstoffe abschwächen, wodurch der Patient gleichzeitig eine Schmerzlinderung erfährt. Dies ist derzeit das Hauptanwendungsgebiet der nichtsteroidalen Entzündungshemmer (Antiphlogistika). Von den Antiphlogistika können nur wenige Vertreter zu differenzierten oder reinen Schmerzbehandlungen eingesetzt werden. Dazu zählen z.B. Indometacin, Naproxen oder Ibuprofen, die auch bei Spasmen der glatten Muskulatur analgetisch wirksam sind. Die weitaus größere Zahl der nichtsteroidalen Antiphlogistika ist wegen nicht ausreichender analgetischer Wirkung und wegen einer Reihe unerwünschter Wirkungen der anti-rheumatischen Therapie vorbehalten.

In US-P 4,927,854 ist ein Arzneimittel beschrieben, welches S(+)-Flurbiprofen, im wesentlichen frei von R(-)-Flurbiprofen enthalten soll. Dieses Mittel wird als Analgetikum, insbesondere für eine verzögerte oder verstärkte Schmerzlinderung empfohlen. Versuche haben jedoch gezeigt, daß S(+)-Flurbiprofen keine direkte schmerzlindernde Wirkung besitzt, sondern nur über eine entzündungshemmende Wirkung die indirekt auch die begleitenden Scherzen abklingen läßt, während das R(-)-Flurbiprofen, welches gemäß der Vorliteratur wirkungslos sein sollte, eine direkte schmerzlindernde Wirkung besitzt.

Die Erfindung hat sich nun zur Aufgabe gestellt, gut einnehmbare oder problemlos parenteral verabreichbare Arzneimittel zur Verfügung zu stellen, welche bei Schmerzen und/oder Entzündungen wirksam sind und eine möglichst niedrige Nebenwirkungsquote besitzen. Ferner sollen solche Arzneimittel einfach herstellbar sein, rasch bzw. kontrolliert anfluten, sich durch gute Bioverfügbarkeit auszeichnen sowie bei Krankheiten mit unterschiedlichen Analgesie- und/oder Antiphlogese-Bedarf durch einfache Variation an die häufig vorkommenden Krankheitsbilder adaptieren lassen.

Diese Aufgabe läßt sich überraschenderweise durch die im Hauptanspruch wiedergegebenen Merkmale lösen. Durch die in den Unteransprüchen gekennzeichneten Maßnahmen wird diese Wirkung noch gesteigert.

Die Enantiomeren werden danach in einem für den jeweiligen Anwendungsfall angepaßten Verhältnis zusammen mit geeigneten pharmakologisch verträglichen Hilfs- und Trägerstoffen zu den erfindungsgemäßen Arzneimitteln verarbeitet.

Bekannte, unerwünschte Nebenwirkungen, wie z.B. Magen-Darm-Beschwerden sind bei nicht steroidalen Entzündungshemmern mit dem Wirkungsmechanismus weitgehend gekoppelt. Diese werden bei der Applikation bei Erkrankungen des rheumatischen Formenkreises in der Medizin in Kauf genommen. Bei Patienten, die jedoch primär einer Schmerzlinderung bedürfen, soll die Quote unerwünschter Wirkungen minimal sein. Dies kann bei Flurbiprofen-Applikation erfindungsgemäß dadurch erreicht werden, daß der Anteil an R(-)-Flurbiprofen in der Enantiomerkombination erhöht wird bzw. das R(-)-Flurbiprofen in reiner Form appliziert wird, da das R(-)-Flurbiprofen einerseits, wie oben gesagt, eine stärkere schmerzlindernde Wirkung im Akutfall hat als das S-Enantiomere und andererseits auch eine geringgradigere Toxizität auf den Gastrointestinaltrakt entfaltet als das Razemat bzw. das S-Enantiomere. Ein Gehalt von 60-99,5 %, vorzugsweise 60-95 %, R(-)-Flurbiprofen und 40-0,5 %, vorzugsweise 40-5 %, S(+)-Flurbiprofen im Wirkstoff ist für die Schmerzlinderung vorgesehen.

Für die Behandlung von entzündlichen Erkrankungen wird dagegen überwiegend das S(+)-Flurbiprofen erwünscht, so daß dabei der Wirkstoff zu 60-99,5 %, vorzugsweise 60-95 % aus S(+)-Flubiprofen und zu 40-0,5 %, vorzugsweise 40-5 % aus R(-)-Flubiprofen besteht.

Die Enantiomeren werden entweder aus Flurbiprofen-Razemat in an sich bekannter Weise durch Razematspaltung isoliert oder durch stereospezifische Synthese hergestellt.

Überraschenderweise zeigt sich, daß die erfindungsgemäß zuvor separierten und dann in gewünschtem Mischungsverhältnis in Fertigarzneimittel wieder zusammengefügten Enantiomeren eine wesentlich schnellere Wirkstoff-Freisetzung aufweisen als das Razemat. Für die therapeutische Anwendung ist dieser Befund bedeutsam, da die raschere Wirkstoff-Freisetzung auch eine entsprechend schnellere Anflutung des Wirkstoffs im Körper zur Folge hat, was gerade bei Schmerzmitteln außerordentlich wichtig ist.

Die Auflösungsgeschwindigkeit wurde dabei gemäß der Vorschrift der USP XXII, S. 683 für Ibuprofen-Tabletten bestimmt (0,9 I Phosphatpuffer pH 7,2, Rührgeschwindigkeit 150 upm, Konzentrationsbestimmung durch UV-Absorptionsmessung bei 220 nm, Mittlung über 10 Tabletten). Verwendet wurden Flurbiprofen-Tabletten mit 100 mg Wirkstoff gemäß Beispiel auf Seite 13. Die Ergebnisse sind in der Graphik gemäß Figur 5 wiedergegeben.

Arzneimittel mit Flurbiprofen werden üblicherweise an Mensch und Tier in Form von Tabletten, Dragees oder Pulver, Granulat, Suppositorien sowie als sterile Lösung parenteral oder als nichtsterile Lösung oder Suspension oral verabreicht. Üblicherweise ist ein rascher Wirkungseintritt gewünscht, jedoch lassen sich auch Darreichungsformen mit verzögerter Freisetzung herstellen, durch die eine länger anhaltende Wirkung gewährleistet ist. Solche Darreichungsformen mit verzögerter Freisetzung sind vorzugsweise solche, welche erst im distalen Darmabschnitt wie im Colon, d.h. verzögert nach Einnahme, dann aber spontan, freigesetzt werden. Eine solche "evening before pill" kann der Patient mit rheumatischen Beschwerden wie Morgensteifigkeit am Abend einnehmen, um erfindungsgemäß am nächsten Morgen beschwerdefrei aufzuwachen. Die bekannten Rezepturen für razemisches Flurbiprofen können ohne weitere Abänderung direkt auch für die erfindungsgemäße Enantiomeren-Mischung verwendet werden.

Bevorzugt wird insbesondere eine orale Verabreichung in Form von Tabletten, Dragees oder Kapseln oder gegebenenfalls auch Kautabletten oder Kaumassen, wobei die pulverförmigen Wirkstoffe in üblicher Weise mit geeigneter Teilchenverteilung mit den bekannten pharmazeutisch verträglichen Hilfs- und Trägerstoffen vermischt und zu Tabletten oder Dragees weiterverpreßt werden bzw. in Gelatine-Kapseln abgefüllt werden.

Die Flurbiprofen-Enantiomere sind in den erfindungsgemäßen Arzneimitteln je nach Darreichungsform in einer Menge von 2 bis 60 % der Rezeptur enthalten.

Feste Darreichungsformen enthalten 20 bis 80 % Füllstoffe. Als solche können unter anderem Stärke, Lactose, Glukose, Mannit, Calciumcarbonat, Calciumphosphat, Cellulose und andere, für diesen Zweck in der Technik bekannte Produkte verwendet werden. Um die Freisetzung zu beschleunigen und damit die Verfügbarkeit zu verbessern, wird der Rezeptur ein Sprengmittel in einer Menge von 2 bis 10 % zugefügt. Als Sprengmittel haben sich insbesondere Carboxymethylstärke, Carboxymethylcellulose, Polyvinylpyrrolidon und Kieselgel bewährt. Darüber hinaus kann die Rezeptur noch Gleitmittel in einer Menge von 0 bis 5 % enthalten, wobei sich Talkum, Magnesium- oder Calciumstearat bzw. andere Hilfstoffe mit Gleiteigenschaften dem Pulver zugesetzt werden, um die Verarbeitung zu erleichtern.

Die Pulver werden üblicherweise trocken gemischt und anschließend mit einem üblichen Bindemittel, beispielsweise Stärkekleister oder auch Wasser feucht granuliert und getrocknet. Das Granulat kann dann anschließend gegebenenfalls unter Zusatz von weiteren Gleitmitteln zu Tabletten verpreßt oder in Kapseln angefüllt werden. Vorteilhaft ist es, Tabletten anschließend noch mit einem Zuckerüberzug zu dragieren oder mit einem löslichen Filmbildner zu lackieren, wobei dieser Überzug zur Verbesserung der Applikationen noch Geschmacks- und Süßstoffe enthalten kann. Neben den in der pharmazeutischen Technik üblichen Überzugsmitteln (Zucker, wie Saccharose oder Laktose, verschiedene Zellulosetypen, wie Methylcellulose oder Celluloseacetatphthalat, Polyacrylaten, Polymethacrylaten oder Polyvinylacetatphthalat) kann vorzugsweise Carnaubawachs als Poliermittel verwendet werden.

Die Abfüllung in Kapseln kann entweder als trockenes Pulver oder Granulat oder Pellets oder als Suspension in einem pflanzlichen Öl oder anderen pharmazeutisch verträglichen flüssigen Trägerstoff erfolgen. Die in Wasser relativ schwer löslichen Wirkstoffe können auch in Gegenwart eines geeigneten Suspensionsmittels wie Traganth, Methylcellulose etc. in Wasser suspendiert werden.

Bekannt ist auch der Einsatz der Flurbiprofen-Wirkstoffe in Form von Suppositorien für die rektale oder vaginale Darreichung, wobei neben dem Wirkstoff Fette oder Polyglykole als Trägerstoffe verwendet werden können, deren Schmelzpunkte entweder im Körpertemperaturbereich liegen, oder die sich nach der Applikation auflösen.

Die Lösungsgeschwindigkeit läßt sich weiterhin auch noch dadurch verändern, daß statt des Flurbiprofens dessen Salze eingesetzt werden. Alkali-, Erdalkali-, Ammonium- oder Aminosäuresalze, welche wasserlöslich sind, werden dabei bevorzugt. Komplexsalze mit basischen Aminosäuren können direkt eingesetzt werden, Mischsalze mit neutralen oder sauren Aminosäuren werden vorher in die Alkali-, Erdalkali- oder Ammoniumsalze überführt. Auch die für andere Arzneimittel bekannte Methode, den Wirkstoff auf Aluminiumoxidgele aufzuziehen, läßt sich mit den erfindungsgemäßen Flurbiprofenen durchführen. Die hergestellten Flurbiprofen-Salze können dann in bekannter Weise, wie oben beschrieben, weiterverarbeitet werden. Vorzugsweise werden die Flurbiprofen-Salze indirekt hergestellt, indem man die zur Salzbildung benötigten Basen der zur Granulierung dienenden Bindemittellösung zufügt, so daß sich die entsprechenden Salze während des Granulierprozesses bilden.

Zur Schmerzbehandlung mit den erfindungsgemäßen Flurbiprofen-Arzneimitteln sind ca. 0,25 bis 5 mg Wirkstoff pro kg Körpergewicht erforderlich, welche beispielsweise in 2 bis 5 Portionen verteilt über den Tag eingenommen werden.
Retardformen wurden insbesondere eingesetzt, um die Applikation auf 1 oder 2 Dosierungen zu reduzieren.
Die Einzeldosis sollte daher zwischen 10 und 100 mg Wirkstoff enthalten.

### Pharmakologische Versuche

### Analgetische Wirkung im Writhing-Test (vgl. Domer, Animal Exp. in Pharm. Analysis, 1971, S. 312)

Je zur Hälfte männliche und weibliche NMRI-Mäuse (definierter Stamm) mit einem mittleren Körpergewicht (KG) von ca. 20 g erhalten pro Dosisgruppe entweder 1,0 mg S(+)- oder 1,0 mg R(-)-Flubiprofen pro kg KG oder eine adäquate Menge Placebo (N=6) p.o. appliziert. Etwa 30 min. nach Applikation der Prüfpräparate erhalten die Versuchstiere eine i.p. Injektion einer wässrigen Essigsäure-Lösung in üblicher Konzentration verabreicht. Beobachtet wird das Auftreten oder Ausbleiben typischer Schleifbewegungen im Sequentialverfahren während einer Beobachtungszeit von 30 min.
Die Ergebnisse sind in der Figur 6 wiedergegeben, wobei in der Ordinate die Anzahl der Schleifbewegungen aufgetragen ist. Das Ergebnis ist gegenüber der Kontrolle signifikant bei R-Applikation (p < 0,05 Student's t-Test zweiseitig).

### Wirkung am Carrageenin-induziertem Pfotenödem der Ratte (vgl. Domer, Animal Exp. in Pharm Analysis, 1971, S. 303)

Männlichen Sprague Dawley-Ratten von jeweils 120-150 g KG wurden die Prüfsubstanzen (0,3 mg/kg KG) per os mit der Schlundsonde appliziert. Unmittelbar danach wurde subplantar 0,1 ml einer 1 %-igen Carrageenin-Lösung in die linke Hinterpfote injiziert, um das Ödem zu evozieren. Nach 3 Stunden p.a. wurden die Pfotenvolumina (modifizierte Methode nach Hofrichter) mittels Plethysmometer bestimmt. Durch S(+)-Flurbipofen wird das Ödem um 64 % und durch R(-)-Flurbiprofen nur um 18 % gehemmt (vgl. Figur 4).

### Gastrointestinale Toxizität an der Ratte (vgl. Beck et al., Arch. Toxicology, 1990, S. 210-217)

R(-)-Flubiprofen verursacht deutlich weniger gastrale Ulzerationen nach oralen Gaben von 25 mg Prüfsubstanz pro kg KG am untersuchten nüchternen Rattenkollektiv (N=9) als S(+)- oder razemisches Flurbiprofen. Im Dünndarm wurden nach oraler Verabreichung von 25 mg R(-)-Gaben pro kg KG nach Futteraufnahme keine Läsionen beobachtet, wie den Ergebnissen der Figur 7 zu entnehmen ist.

Die Testgruppen werden jeweils nach 24 Stunden getötet, Magen und Darm entnommen, und der Magen geöffnet und mit Salzlösung gereinigt. Die Zahl der Ulzerationen multipliziert mit ihrem Durchmesser in mm wird als "gastric ulcer index" angegeben. Der Dünndarm wird ungeöffnet auf weiße und braune Farbveränderungen untersucht. Die entsprechenden Abschnitte werden herausgeschnitten und ihr Gewicht im Verhältnis zum Gesamtgewicht als "intestinal ulcer" in % aufgetragen. Die Ergebnisse sind signifikant im t-Test gegenüber der Kontrolle.

### Arzneimittelzusammensetzungen

### Tabletten

Eintausend Tabletten mit jeweils einem Gehalt von 100 mg Flurbiprofen als Pseudorazemat werden wie folgt hergestellt:

| | |
|---|---|
| R(-)-Flurbiprofen | 50 g |
| S(+)-Flurbiprofen | 50 g |
| Laktose | 75 g |
| Maisstärke | 50 g |
| Magnesiumstearat | 4 g |
| Siliziumdioxid | 5 g |

Die Enantiomeren werden fein gemahlen (Luftstrahl-Mühle), mit den Hilfsstoffen gemischt und vorkomprimiert. Daraus wird in bekannter Weise ein Granulat hergestellt, welches zu Tabletten von ca. 235 mg verpreßt wird.

In Anlehnung an diese Herstellungsvorschrift können auch Tabletten mit anderen Enantiomer-Anteilen pro Tablette im beanspruchten Verhältnis hergestellt werden. Ferner lassen sich auf der Basis dieser Zusammensetzung auch Tabletten mit beispielsweise einem Gesamtwirkstoffanteil von 25 oder 50 mg produzieren.

### Injektionslösung

Eine sterile wässrige Lösung für die parenterale Applikation, die pro Liter 350 mg des Enantiomeren-Gemisch enthält, wird beispielhaft als Natrium-Salz wie folgt hergestellt:

| | |
|---|---|
| R(-)-Flurbiprofen Natriumsalz ^{*}) | 266 mg |
| S(+)-Flurbiprofen Natriumsalz ^{*}) | 87 mg |
| Wasser p.i.; q.s. | 1000 ml |

| | |
|---|---|
| ^{*}) 99,5 % Reinheit | |

Anstelle der Natriumsalze können auch andere Salze, die nach Neutralisation der enantiomerreinen Wirkstoffen mit beispielsweise Ammoniak, Aminosäuren wie Lysin etc. erhalten und unter Berüchsichtigung der jeweiligen Äquivalentgewichte eingesetzt werden. Die Lösungen werden in sterile Behältnisse filtriert und verschlossen.

### Hartgelatine-Kapseln

Etwa 1000 Hartgelatine-Kapseln mit 50 mg R(-)-Flurbiprofen für die orale Verabreichung werden wie folgt hergestellt:

| | |
|---|---|
| R(-)-Flurbiprofen | 50 g |
| Laktose | 100 g |
| Maisstärke | 20 g |
| Talkum | 20 g |
| Magnesiumstearat | 2 g |

Das fein gemahlene R(-)-Flurbiprofen wird mit den anderen Ingredientien homogen gemischt und in bekannter Weise in Kapseln abgefüllt. Analog können Kapseln mit 25, 75 oder 100 mg R(-)-Flurbiprofen aber auch mit Flurbiprofen-Enantiomer-Gemischen im beanspruchten Verhältnis hergestellt werden.

### Suspension zur oralen Einahme

Um 1000 ml einer wässrigen Suspension herzustellen, wobei eine orale Dosiseinheit (1 Teelöffel - 5 ml) 5 mg R(-)-sowie 95 mg S(+)-Flurbiprofen als Aluminium-Salze enthält, wird von folgender Zusammensetzung ausgegangen:

| | |
|---|---|
| R(-)-Flurbiprofen | 1 g |
| S(+)-Flurbiprofen | 19 g |
| Zitronensäure | 2 g |
| Benzoesäure | 1 g |
| Zucker | 700 g |
| Tragacanth | 5 g |
| Zitronenöl | 2 g |
| Wasser, entsalzen; q.s. | 1000 ml |

Zunächst werden Zitronensäure, Benzoesäure, Zucker, Tragacanth und Zitronenöl mit soviel Wasser suspendiert, damit etwa 800 bis 900 ml Suspension anfallen. Danach werden die mikronisierten Flurbiprofen-Enantiomeren homogen eingerührt sowie auf 1000 ml mit Wasser aufgefüllt.

### Suppositorien

Ein Suppositorium, das als Wirkstoffe 10 bis 100 mg des Enantiomeren-Gemisches enthalten kann und ca. 2 g wiegt, ist wie folgt zusammengesetzt:

| | |
|---|---|
| R(-)-Flurbiprofen | 90 mg |
| S(+)-Flurbiprofen | 10 mg |
| Hartfett | 1890 mg |
| Tocopherol | 10 mg |

Falls der/die Wirkstoffanteil(e) reduziert wird/werden, ist im selben Ausmaß der Hartfettanteil zu erhöhen.

### Creme

Die Herstellung einer Creme mit 4% Flurbiprofen-Enantiomeren erfolgt in an sich bekannter Weise, wobei folgende Komponenten eine typische Rezeptur ergeben:

| | |
|---|---|
| R(-)-Flurbiprofen | 1,0 g |
| S(+)-Flurbiprofen | 3,0 g |
| Triglyzende; mittelkettig | 35,0 g |
| Glyzennmonostearat-Polyoxyethylenstearat-Gemisch | 6,0 g |
| Polyoxyethylen-Fettsäureester | 4,0 g |
| 1,2-Propandiol | 4,0 g |
| 4-Hydroxybenzoesäuremethylester-Natrium | 0,1 g |
| Xanthan-Gummi | 0,2 g |
| Wasser, entsalzen; q.s. | 100,0 g |

In der auf ca. 60 °C erwärmten öligen Phase werden die Wirkstoffe gelöst, die ebenfalls vorerwärmte wässrige Phase wird danach eingerührt und bis zur Abkühlung gleichmäßig weitergerührt. Ein Strang von ca. 2.5 cm enthält etwa 100 mg des Wirkstoff-Gemisches.

### Filmtablette

### Beispiele einer Flurbiprofen-Tablettenrezeptur

38,5 kg Flurbiprofen-Lysinat wurden trocken mit 7,5 kg mikrokrist. Cellulose gemischt, mit 3 kg Gelatine (10 %-ig in Wasser) granuliert und getrocknet, mit 0,5 kg Mg-stearat, 1 kg Talcum und 2 kg Na-carboxymethylcellulose vermischt und zu runden Tabletten mit einem Durchmesser von 6 mm und einem Gewicht (Restfeuchte: 0,8 - 1,5 %) von 260 mg verpreßt. Die fertigen Tabletten werden mit einem Lacküberzug aus einer Lösung aus 0,7 % Glycerin, 4 % Methylcellulose, 0,7 % Polyglycol 6000, 58 % Wasser und 36,6 % Aceton überzogen und getrocknet.

Eingesetzt wurde dabei:
R(-)-Flurbiprofen im wesentlichen frei von S(+)-Flurbiprofen, S(+)-Flurbiprofen im wesentlichen frei von R(-)-Flurbiprofen und
ein Pseudoracemat aus 50 % S(+)- und 50 % R(-)-Flurbiprofen.

## Patentansprüche

1. Verwendung von Flurbiprofen zur Herstellung eines Arzneimittels zur Behandlung von schmerzhaften Erkrankungen, **dadurch gekennzeichnet, daß** bei Schmerzen oder bei chronischen Krankheitsbildern mit dominierenden Schmerzzuständen 60 bis 99,5 %, vorzugsweise 60 bis 95 %, R(-)-Flurbiprofen, Rest S(+)-Flurbiprofen verwendet wird, wobei die zuvor separierten Enantiomere des Flurbiprofens im gewünschten Verhältnis rekombiniert und mit üblichen festen pharmazeutischen Trägern und Hilfsstoffen zu Arzneimitteln verarbeitet werden.

2. Verwendung nach Anspruch 1, **dadurch gekennzeichnet, daß** das Arzneimittel oral als Tabletten, Dragees, Kaumasse, Suspensionen, anal als Suppositoren oder parenteral intramuskulär als Suspensionen verwendet wird.

3. Verwendung nach den Ansprüchen 1 oder 2, **dadurch gekennzeichnet, daß** das Flurbiprofen als Alkali, Erdalkali, Ammonium, Aminosäuresalz - vorzugsweise als Lysinat - oder Aluminiumverbindung, gegebenenfalls als Mischung, insbesondere der verschiedenen Enantiomeren, verwendet wird.

4. Verwendung nach den Ansprüchen 1 bis 3, **dadurch gekennzeichnet, daß** die Mischung retardierend wirkende Zusätze oder Überzüge enthält.

## Claims

1. The use of flurbiprofen in the manufacture of medicaments for the treatment of painful diseases, **characterised in that** the flurbiprofen in the case of pains or in the case of chronic disease pictures with dominating states of pain is used as 60 to 99.5 %, preferably 60 to 95 % R(-)-flurbiprofen, remainder S(+)-flurbiprofen, whereby the previously separated enantiomers of the flurbiprofen are recombined in the desired ratio and worked up with usual solid pharmaceutical carriers and adjuvant materials to give medicaments.

2. The use according to claim 1 **characterised in that** the medicament is administered orally as tablets, coated tablets, chewing masses, suspensions, anally as suppositories or parenterally or intramuscularly as suspensions.

3. The use according to claim 1 or 2, **characterised in that** the flurbiprofen is used as alkali metal, alkaline earth metal, ammonium amino acid salt - preferably as lysinate - or aluminium compound, possibly as mixture especially of the different enantiomers.

4. The use according to claims 1 to 3 **characterised in that** the mixture contains retarding-acting addititves or coatings.

## Revendications

1. Utilisation de flurbiprofène pour la préparation d'un médicament destiné au traitement de maladies douloureuses, **caractérisée en ce que**, dans le cas de douleurs ou dans le cardre de tableaux cliniques chroniques présentant des états douloureux dominants, on utilise, à concurrence de 60 à 99,5 %, de préférence de 60 à 95 %, du R(-)flurbiprofène, le reste étant le S(+)-flurbioprofène et qu'on recombine les énantiomères du flurbiprofène séparés au préalable, dans un rapport désiré, et on les traite avec des supports et des adjuvants pharmaceutiques solides habituels pour obtenier des médicaments.

2. Utilisation selon la revendication 1, **caractérisée en ce qu'**on utilise le médicament par voie orale sous forme de comprimés, de dragées, de matière à mastiquer, de suspensions, par voie anale sous forme de suppositoires ou encore par voie parentérale ou par voie intramusculaire sous forme de suspensions.

3. Utilisation selon les revendications 1 ou 2, **caractérisée en ce qu'**on utilise le flurbiprofène sous forme de sels de métaux alcalins, de métaux alcalino-terreux, d'ammonium, d'aminoacides - de préférence sous forme de lysinate - ou encore sous forme d'un composé d'aluminium, éventuellement sous forme d'un mélange, en particulier des différents énantiomères.

4. Utilisation selon les revendications 1 à 3, **caractérisée en ce que** le mélange contient des revêtements ou des additifs à effet retard.
